(19) Europäisches Patentamt
European Patent Office
Office européen des brevets

(11) Publication number: **0 173 944**
**B1**

(12) **EUROPEAN PATENT SPECIFICATION**

(45) Date of publication of patent specification: **05.04.89**

(51) Int. Cl.$^4$: **C 12 Q 1/04** // C12Q1/18, C12N1/38, C12N1/16

(21) Application number: **85110722.7**

(22) Date of filing: **26.08.85**

(54) **Enhanced inhibition of yeast cell growth.**

(30) Priority: **07.09.84 US 648706**

(43) Date of publication of application:
**12.03.86 Bulletin 86/11**

(45) Publication of the grant of the patent:
**05.04.89 Bulletin 89/14**

(84) Designated Contracting States:
**AT CH DE FR GB IT LI NL SE**

(56) References cited:
**GB-A-2 128 617**

**NATURE, vol. 303, 9th June 1983, pages 526-528, Basingstoke, GB; M. VAARA et al.: "Sensitization of gram-negative bacteria to antibiotics and complement by a nontoxic oligopeptide"**

**CHEMICAL ABSTRACT, vol. 80, no. 11, 18 March 1974, Columbus, Ohio (US); S.CHIHARA et al.: "Enzymatic degradation of colistin. Isolation and identification of alpha-N-acyl alpha,gamma-diaminobutyric acid and colistion nonapeptide", p. 100, no. 56732j**

(73) Proprietor: **MILES INC.**
**1127 Myrtle Street**
**Elkhart Indiana 46514 (US)**

(72) Inventor: **Boguslawski, George**
**2323 Kenilworth Drive**
**Elkhart, IN 46514 (US)**

(74) Representative: **Dänner, Klaus, Dr. et al**
**c/o Bayer AG Konzernverwaltung RP**
**Patentabteilung**
**D-5090 Leverkusen 1 Bayerwerk (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

I. Field of the Invention

Polymyxin B is a complex antibiotic substance elaborated by various strains of *Bacillus polymyxa* and is toxic to bacterial, fungal and animal cells. This toxicity is attributable to the ability of the compound to disrupt the integrity of cell membranes thereby allowing cell contents to escape. The antibiotic consists of a cyclic peptide portion and a fatty acyl portion joined by an amide bond. This amide bond can be cleaved by papain or ficin resulting in a cyclic nonapeptide referred to as polymyxin B nonapeptide or simply PBN. PBN has been shown to be nontoxic to bacteria but sensitizes the bacterial cells to other antibiotics or serum complement.

II. Description of Pertinent Art

Vaara and Vaara (*Nature,* Vol. 303, 526—528, 1983) diclose that PBN (described therein as PMBN) may be used to increase the permeability of the outer cell membranes of gram-negative bacteria thereby sensitizing gram-negative enteric bacteria to complement and antibiotics. The bacteria tested were clinical isolates of *E. coli, Klebsiella pneumoniae, Klebsiella oyxtoca, Enterobacter agglomerans, Salmonella typhimurium,* and *Pseudomonas aeruginosa.* The authors indicate that because of the increased sensitivity of the cells to antibiotics, PBN could be used clinically to extend the antibacterial spectrum of many antibiotics. No description or suggestion of the use of PBN in lower eucaryotes, such as yeast, is given. UK Patent Application GB 2,128,617A makes a substantially similar disclosure.

Storm, et al (*Ann. Rev. Biochem., 46,* 723—763, 1977) provide a review of the biochemical and physiological data of the polymyxin antibiotics and the effects thereof on bacterial cells.

None of the above references describe or suggest the use of PBN to increase the inhibition of growth of yeast by various antibiotic substances.

Summary of the Invention

The present invention is directed to a method of increasing the inhibition of growth of yeast cells by antibiotics. The method provides for incubating the yeast cells with an effective amount of polymyxin B nonapeptide in the presence of a growth inhibiting amount of an antibiotic selected from the group consisting of rifampicin, erythromycin, G—418, cycloheximide and actinomycin D. Contacting said yeast cells with polymyxin B nonapeptide is believed to increase the permeability of said cells, thereby increasing the inhibition of growth thereof by antibiotics to which the cells are sensitive or may otherwise be resistant.

The designation G—418 as used herein refers to the antibiotic Geneticin® available from Gibco Laboratories.

Detailed Description of the Invention

Polymyxin B nonapeptide (alternatively referred to herein as "PBN") is prepared from the antibiotic polymyxin B by the enzymatic cleavage of the amide bond joining the fatty acyl moiety and the peptide moiety of the molecule. The reaction may be illustrated as follows (where "dab" refers to diaminobutyric acid and "FA" refers to fatty acid):

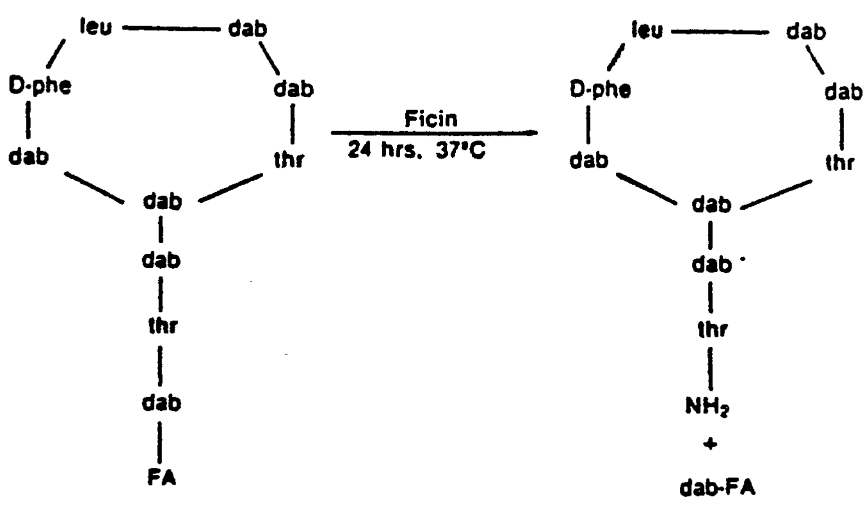


The preparation of PBN is accomplished by the method of Chihara, et al as described in *Agr. Biol. Chem.,* 37(11), 2455—2463 (1973) which is incorporated herein by reference. Polymyxin B sulfate is hydrolyzed by purified ficin in a phosphate buffer (pH 7) for 24 hours at 37°C. The reaction mixture is then

boiled (to coagulate the enzyme) and the polymyxin B hydrolysate is clarified by filtration. The clarified solution is then acidified to pH 2 with 1 normal (N) HCl and subseuqently extracted wit n-butanol to dissolve fatty acyl diaminobutyric acid. The residual aqueous solution is adjusted to pH 8 with 1N NaOH and is repeatedly extracted with n-butanol to remove any remaining polymyxin B. The pH of the aqueous layer is again adjusted (i.e., pH 7) and the material is passed through an Amberlite® IRA—410 column (OH type). The effluent is adjusted to pH 5, concentrated in vacuo and lyophilized. Because the resulting PBN contains large amounts of NaCl (3—4 M), the salt may be removed by standard techniques such as filtration, for instance, through a Sephadex® G10 column using ammonium carbonate as the eluent.

The method of the invention provides for incubating yeast cells with an effective amount of PBN in the presence of a growth inhibiting amount of an antibiotic substance. For these purposes, the amount of PBN to be utilized (i.e., the "effective amount") may range anywhere from about 1 to about 10 $A_{260}$ units. One $A_{260}$ unit refers to the absorbance at 260 nanometers of a 1 milliliter (ml) sample of PBN. One unit is equal to approximately 6 milligrams of salt-free PBN. It is believed that the absorbance measurement of PBN is a more reliable method than a determination by weight of the concentration of PBN in a sample due to the quantity of salt present following the enzymatic hydrolysis of polymyxin B sulfate. The incubation of the yeast cells with the desired amount of PBN may be carried out in any convenient manner in the presence of a growth inhibiting amount of antibiotic. As used herein, the term "growth inhibiting amount" refers to that amount of antibiotic necessary to suppress the growth of the yeast cells which have been contacted with PBN. The exact quantity of antibiotic to be used in a given situation will depend on factors such as the antibiotic being used, the species and/or strain of yeast being tested and the like. In any event, that amount of antibiotic may be readily determined by the skilled artisan utilizing conentional techniques. It should be pointed out that the use of PBN as described herein will either serve to enahnce the sensitivity of yeast cells to antibiotics to which the cells are previously known to be sensitive, or the PBN may render cells sensitive to antibiotics to which the yeast is normally resistant. However, the effect of PBN described herein is selective in that PBN does not cause an increase in yeast cell growth inhibition by all antibiotic substances.

A convenient method for ascertaining the inhibition of growth of yeast cells is as follows. A sample of yeast cells (about $10^7$ cells) from a viable overnight culture is added to a molten agar-containing growth medium to which has been added the desired amount of PBN. The resulting suspension is mixed rapidly and then poured onto an agar plate containing a suitable growth medium. The molten agar is allowed to solidify and sterile filter discs are placed on the surface. The solution of antibiotics to be tested are then pipetted onto the discs (50 microliters per disc). The plates are incubated and "read" for antibiotic sensitivity as determined by the zone of inhibition around the disc.

The yeasts to which the method of the present invention are applicable include, but are not limited to, various strains of *Saccharomyces cerevisiae,* various *Candida* species including *Candida albicans, Candida tropicalis, Candida stellatoidea* and the like; *Crytococcus neoformans, Coccidioides immitis, Torulopsis glabrata, Histoplasma capsulatum* and *Trichosporon cutaneum.*

The following examples are provided as a means of illustrating the present invention and are not to be construed as a limitation thereon.

## Example I

The effect of PBN on the Inhibition of Yeast Cell Growth by Various Antibiotics

A. Preparation of Polymyxin B Nonapeptide

Polymyxin B nonapeptide (PBN) was prepared from polymyxin B sulfate (Sigma Chemical Co., catalogue number P1004) according to the procedure of Chihara, et al, supra. 9 grams (g) of polymyxin B sulfate was dissolved in 900 ml of a 0.1 M sodium phosphate buffer (pH 7). To this was added 2000 units of ficin (available from Sigma Chemical Co., catalogue number 4125) and the resulting mixture was maintained at 37°C for 24 hours with gentle agitation. The mixture was then boiled for 15 minutes to coagulate the enzyme and was then filtered through a 0.45 μm pore size filter. The clarified solution was acidified to pH 2 with 1N HCl and then extracted with n-butanol. The residual aqueous solution was adjusted to pH 8 with 1N NaOH and was again extracted with n-butanol. The aqueous layer was adjusted to pH 7 after which it was passed through an Amberlite® IRA—410 column (OH-type). The effluent was then lyophilized and dissolved in a total of 37 ml $H_2O$. The resulting solution had a concentration of 12.5 $A_{260}$ units. A 10 ml sample of this solution was desalted on a Sephadex® G10 column using ammonium carbonate as the eluent. The effluent was again lyophilized and dissolved in 5 ml of $H_2O$ giving the final solution a concentration of 15.2 $A_{260}$ units.

B. The Effect of Polymyxin B Nonapeptide on *Saccharomyces cerevisiae*

*Saccharomyces cerevisiae* strain S288C was grown for 16 hours at 30°C in a liquid medium consisting of 1% (w/v) yeast extract, 2% (w/v) peptone, and 2% (w/v) glucose (YPD medium). One drop of cell suspension from this culture (about $5 \times 10^6$ to about $1 \times 10^7$ cells) was added to each of 2 tubes containing 3 ml of 0.7% agar, 1% yeast extract, 2% peptone, 2% glycerol (or alternatively 2% glucose as a carbon source) and 0.25 ml of 5 M NaCl maintained at 45—50°C to prevent the agar from solidifying. One tube contained, in addition to the above, 0.5 ml of PBN solution (4.8 $A_{260}$ units) prepared as described above.

The other tube served as a control. The tube contents were mixed rapidly and poured onto a Petri plate containing 25 ml of 2% agar, 1% yeast extract, 2% peptone, and 2% glycerol (YPG medium). After the agar had solidified, sterile filter disks (0.5 inch diameter) were placed on the surface of the agar and solutions of various antibiotics to be tested were pipetted onto the disks. The plates were incubated at 30°C for 48 hours and the inhibition of growth was determined by measuring the area of a zone of clearing formed around the discs (minus the area of the disc). The results are shown in Table 1.

TABLE 1
The Effect of PBN on the Inhibition of Yeast Growth in YPG Medium by Various Antibiotics

| Antibiotic (mg/disk) | Area of Zone of Inhibition ($mm^2$) Control | PBN ($4.8\ A_{260}$ units) |
|---|---|---|
| Erythromycin (0.5)[a] | 80 | 227 |
| G—418 (5.0)[b] | 113 | 490 |
| Rifampicin (2.5)[c] | <3 | 95 |
| Actinomycin D (0.5)[c,d] | <3 | 314 |

[a]In 30% ethanol
[b]G—418 refers throughout to the antibiotic Geneticin® available from Gibco Laboratories
[c]In dimethylsulfoxide
[d]YPD medium used for plating

These results suggest that PBN facilities uptake of the above antibiotics by yeast cells. Erythromycin, which affects mitochondrial protein synthesis, is effective in this test system only when glycerol (rather than glucose) is used as the carbon source. This is due to the fact that mitochondrial protein function is not required by the cells when glucose is used as the carbon source. Rifampicin, G—418 and actinomycin D exhibited inhibition of yeast growth regardless of the carbon source, while kanamycin, streptomycin and chloramphenicol (5.0, 2.5 and 0.25 mg/disc, respectively) exhibited no effect in this experiment for both the control and PBN-containing samples (data not shown in Table 1).

Example II

The Effect of PBN on the Growth of *Candida albicans*

The procedure described in Example I was repeated with *Candida albicans (ATCC 10231).* Inhibition of growth of this organism by G—418, rifampicin, cycloheximide and actinomycin D in the presence and absence (control) of PBN was determined. The results are set forth in Table 2.

TABLE 2
The Effect of PBN on the Growth of *Candida albicans* in the Presence of Various Antibiotics

| Antibiotic (mg/disk) | Area of Zone of Inhibition ($mm^2$) Control | PBN[a] |
|---|---|---|
| G—418 (5.0) | 20 | 346 |
| Rifampicin (2.5) | <3 | 3 |
| Cycloheximide (0.5) | <3 | 64 |
| Actinomycin D (0.5) | <3 | 452 |

[a]PBN present in a concentration of $3.0\ A_{260}$ units per plate in YPD medium.

These data show that in the presence of PBN, growth of *C. albicans* strain ATCC 10231 is inhibited by antibiotics to which the organism is otherwise resistant or only weakly sensitive. This observation suggests that PBN may be useful as a therapeutic adjunct in the treatment of various *Candida* infections of the skin, mucosa and intestinal tract as well as other infections of yeast origin.

Example III

The Effect of Varying Concentration of PBN on Inhibition of Yeast Growth by Rifampicin

The procedure described in Example I was repeated with *Saccharomyces cerevisiae* strain S288C except that increasing amounts of PBN were used. The rifampicin concentration of the discs was maintained at 2.5 mg/disc throughout. The results are shown in Table 3.

TABLE 3

The Effect of Increasing Amounts of PBN on Growth Inhibition by Rifampicin

| PBN Concentration ($A_{260}$ units) | Area of Zone of Inhibition ($mm^2$) |
|---|---|
| 0.0 | 0 |
| 1.0 | 3.0 |
| 2.0 | 20.0 |
| 3.0 | 38.5 |
| 4.0 | 38.5 |
| 5.0 | 50.0 |
| 6.0 | 64.0 |
| 7.0 | 78.5 |
| 8.0 | 71.0 |
| 9.0 | 78.5 |
| 10.0 | 95.0 |

These data show that the increase in growth inhibition by rifampicin is approximately proportional to the concentration of PBN used.

**Claims**

1. A method of increasing the inhibition of growth of yeast cells which comprises incubating the yeast cells with an effective amount of polymyxin B nonapeptide in the presence of a growth inhibiting amount of an antibiotic selected from the group consisting of rifampicin, erythromycin, G—418, cycloheximide and actinomycin D.

2. The method of claim 1 wherein the yeast is a species of *Candida* or *Saccharomyces.*

3. The method of claim 2 wherein the yeast is *Candida albicans.*

**Patentansprüche**

1. Verfahren zur Erhöhung der Hemmung des Wachstums von Hefezellen, gekennzeichnet durch Inkubieren der Hefezellen mit einer wirksamen Menge von Polymyxin-B-nonapeptid in Gegenwart einer wachstumsinhibierenden Menge eines Antibiotikums, ausgewählt aus der Gruppe, bestehend aus Rifampicin, Erythromycin, G—418, Cycloheximid un Actinomycin D.

2. Verfahren nach Anspruch 1, worin die Hefe eine Spezies von Candida oder Saccharomyces darstellt.

3. Verfahren nach Anspruch 2, worin die Hefe Candida albicans darstellt.

**Revendications**

1. Un procédé pour augmenter l'inhibition de la croissance des cellules de levures, qui consiste à incuber les cellules de levures avec une quantité efficace de nonapeptide de polymyxine B en présence d'une quantité d'un antibiotique inhibant la croissance, choisi parmi la rifampicine, l'érythromycine, la G—148, le cycloheximide et l'actionmycine D.

2. Le procédé selon la revendication 1, dans lequel la levure est une espèce de Candida ou de Saccharomyces.

3. Le procédé selon la revendication 2, dans lequel la leveure est Candida albicans.